# EUROPEAN PATENT SPECIFICATION

(11) **EP 3 116 580 B1**
(45) Date of publication and mention of the grant of the patent: **18.04.2018**
(21) Application number: 15717624.9
(22) Date of filing: 06.03.2015
(51) Int. Cl.: A61M 25/00, A61M 25/06, A61M 39/06, A61M 39/22, A61M 39/28

(54) **AN ANTI-REFLUX CATHETER**
KATHETER MIT RÜCKLAUFSCHUTZ
CATHÉTER ANTI-REFLUX

(30) Priority: 10.03.2014 IT MO20140059
(43) Date of publication of application: 18.01.2017
(73) Proprietor: Delta Med S.P.A. Unipersonale, 46019 Viadana (Mantova) (IT)
(72) Inventor: BERTOLI, Alessandro, I-26037 San Giovanni In Croce (cr) (IT); BALBONI, Alessandro, I-46030 San Giorgio di Mantova (MN) (IT)
(74) Representative: Bergamini, Silvio
(86) International application number: PCT/IB2015/051666
(87) International publication number: WO 2015/136423

(56) References cited:
- EP-A1- 0 502 321
- WO-A1-96/26753
- WO-A1-2004/009171
- WO-A1-2005/011800
- US-A- 4 960 259

## Description

### Field of the invention

The invention relates to an anti-reflux catheter, which is generally designed to be introduced into a blood vessel of a patient using a coaxially-received introducer needle, and can block outward blood refluxes when the introducer needle is removed from the catheter.

### Background art

Flexible catheters are known to be designed to be introduced into a blood vessel of a patient using an introducer needle, which is coaxially introduced into the lumen of the catheter before injection, to impart such a relative temporary axial stiffness thereto, as to allow it to penetrate the blood vessel without bending.

Typically, a catheter adapted to be introduced into a blood vessel comprises a flexible tubular body which has, at one end designed as a proximal end, i.e. facing an operator during use, a support element, known and referenced hereibelow as "hub", which has an inner axial passage cavity, seamlessly connected to the tubular body and, in certain examples also a pair of wings bilaterally extending from the hub.

The latter defines an end facing away from that with the tubular body attached thereto, which forms an opening having a peripheral standard connection profile for tubing or other medical equipment.

Typically, the hub also has a so-called transverse "injection point" thereon, consisting of a small cylindrical dimple that laterally projects from the hub and has an axial channel communicating with the axial inner cavity of the hub.

The cylindrical dimple is typically sealed at its base with an elastically flexible cylinder which is coaxially, stably forced into the axial inner cavity of the hub, and can maintain the seal when it is not subjected to transverse stresses due to the pressure of a liquid which is introduced through the cylindrical dimple, for instance by means of a syringe, and has to be added to the normal flow of medicament that flows in the axial inner cavity of the hub; in this condition, the cylinder partially bends and opens the connection between the axial channel of the dimple and the axial inner cavity of the hub.

In other words, this injection point allows the patients to be administered additional medicaments, when needed, to be mixed with the drugs that normally flow along the tubular body of the catheter, during infusion, into the blood vessel of the patient.

The introducer needle consists of a thin, axially hollow metal shaft having a tip at one end and, at the opposite end, a support element having a finger grip portion for the operators and a connector, also of standard type, for additional tubing or equipment, which is protected by a screw cap when not in use.

The needle shaft is slightly longer than the tubular body of the catheter such that, when it is entirely inserted therein in a ready-for-injection configuration, the tip and a very short distal section of the shaft project out of the corresponding end of the tubular body, to prick and open a passage for the tubular wall in the blood vessel wall.

Once the injection has been made and the catheter has been placed in the right position, the operator fixes the hub to the epidermis of the patient, typically by means of an adhesive strap and holds the hub with one hand while removing the introducer needle from the catheter with the other hand, to release the tubular body in which the medicinal solutions to be infused to the patient are designed to flow. A relevant example can be found in document WO-A-96/26753

This prior art has the drawback that, as soon as the operator has completed the removal of the introducer needle shaft from the tubular body of the catheter, blood refluxes outwards therethrough from the blood vessel of the patient, due to the blood pressure therein.

In order to obviate this drawback and stop blood flowing out of the proximal end of the catheter, the operators can only press the blood vessels with their fingers immediately upstream from the end of the tubular body, until the proximal end of the hub is connected to the connector of an infusion tube.

This step requires a quick action by the operators, which are often incomplete or imperfect, because the tubular body is often pressed, thereby causing some blood outflow, and creating serious hazards of infection transmission by contact for operators, as well as contamination of the surrounding environment, as well as possible panic and anxiety in patients, as they see their own blood.

Furthermore, the required strong pressure on the blood vessel or, mistakingly, on the tubular body of the catheter, may cause pain in the patient due to crushing of an area of the body that has already been pricked for introducing the catheter in the blood vessel, and may be affected by an inherently painful disease.

It shall be noted that the above mentioned problems reiterate each time that different drugs have to be administered to a patient.

Therefore, alternation of different administrations requires the drug infusion flows that have been administered before through respective lines to be stopped, the lines to be removed, and a second line to be attached for administration of a second drug.

These disconnection and reconnection steps must be carried out while pressing the blood vessel of the patient to avoid outward blood refluxes.

### Disclosure of the invention

One object of the invention is to improve the prior art.

Another object of the invention is to provide an anti-reflux catheter that affords easy blocking of outward blood reflux from a blood vessel once the introducer needle has been removed from the tubular body.

A further object of the invention is to provide an anti-reflux catheter that allows health operators to use it with no hazard of infection transmissions by accidental contact with patient blood outflowing from catheters, and to avoid the other above mentioned problems.

Yet another object of the invention is to provide an anti-reflux catheter that can be quickly and simply operated, without causing additional pain to patients.

The invention provides an anti-reflux catheter as defined by the features of claim 1.

The invention achieves the following advantages:
- blocking blood reflux from a blood vessel of a patient once the catheter has been introduced and the introducer needle has been removed therefrom;
- avoiding any accidental contact between patient blood and health operators;
- when needed, clearing the passage in the tubular body of the catheter for the flow of medicaments to be infused or stop them as needed.

### Brief description of the drawings

Further features and advantages of the invention will be more readily apparent upon reading of the detailed description of a preferred non exclusive embodiment of an anti-reflux catheter, which is shown as a non limiting example by the annexed drawings, in which:
FIG. 1 is a perspective view of an anti-reflux catheter of the invention, in which the introducer needle is completely retracted;
FIG. 2 is a side view of the anti-reflux catheter of Figure 1, without the introducer needle;
FIG. 3 is a perspective, enlarged view of a possible example of the anti-reflux catheter;
FIG. 4 is a longitudinal sectional view of the anti-reflux catheter according to the example of Figure 3, in a clear passage state;
FIG. 5 is a cross sectional view of the anti-reflux catheter according to the example of Figure 3, in the same clear passage state;
FIG. 6 is a longitudinal sectional view of the anti-reflux catheter according to the example of Figure 3, in a closed passage state;
FIG. 7 is a cross sectional view of the anti-reflux catheter according to the example of Figure 3, in the same closed passage state;
FIG. 8 is a cross half-sectional view of the catheter of Figure 3, in a clear passage state;
FIG. 9 is a cross half-sectional view of the catheter of Figure 3, in a closed passage state;
FIG. 10 is a longitudinal sectional view of an anti-reflux catheter according to an embodiment, in a clear passage state;
FIG. 11 is the corresponding cross sectional view of the anti-reflux catheter of Figure 10, in the clear passage state;
FIG. 12 is a longitudinal sectional view of the anti-reflux catheter of Figure 10, in a closed passage state;
FIG. 13 is the corresponding cross sectional view of the anti-reflux catheter of Figure 12, in the closed passage state;
FIG. 14 is a perspective, enlarged view of the embodiment of the anti-reflux catheter of Figure 10.

### Detailed description

Referring to the figures, numeral 1 generally designates an anti-reflux catheter of the invention, hereinafter simply catheter 1, which comprises a central body 2 having a substantially tubular, frustoconical shape, and defining therein a chamber 3 for the passage of fluid medicaments to be infused into a blood vessel of a patient

Two wings 4 bilaterally extend from the central body 2, and provide two surfaces for the catheter 1 to be laid and attached to the epidermis of the patient, near its insertion point.

A tubular flexible cannula 5 extends from a distal end 2A of the body 2 and is combined with the body 2 to form the catheter 1.

The end 2B of the body 2 that faces away from the cannula 5 is formed with an opening whose outer contour has a standard connection profile which is designed to be coupled to a mating-profile of a concurrent drug delivery tube, not shown.

A sleeve 7 made of an elastically flexible material is coaxially arranged in the chamber 3, with its outer surface adhering to the inner walls of the chamber 3, and defines a central lumen 12.

A transverse opening 8 is formed on one side of the body 2 to communicate with the inner chamber 3, and has an outwardly projecting dimple-like mouthpiece 9.

Typically, this mouthpiece 9 has a cylindrical shape, defines therein a sliding chamber 9A and is perpendicular to the body 2.

The mouthpiece 9 may be fitted with a closing cap 10, which has a closing member 11 extending from its inner central portion and received in the chamber 9A, to face the sleeve 7 when the cap 10 is mounted to the mouthpiece 9.

The closing member 11 is preferably formed like a pin 11A, and has one end 11B facing away from the cap 10 that extends through the cap 2, passing through the transverse opening 8, such that in certain conditions it may contact the outer surface of the sleeve 7, as better explained below.

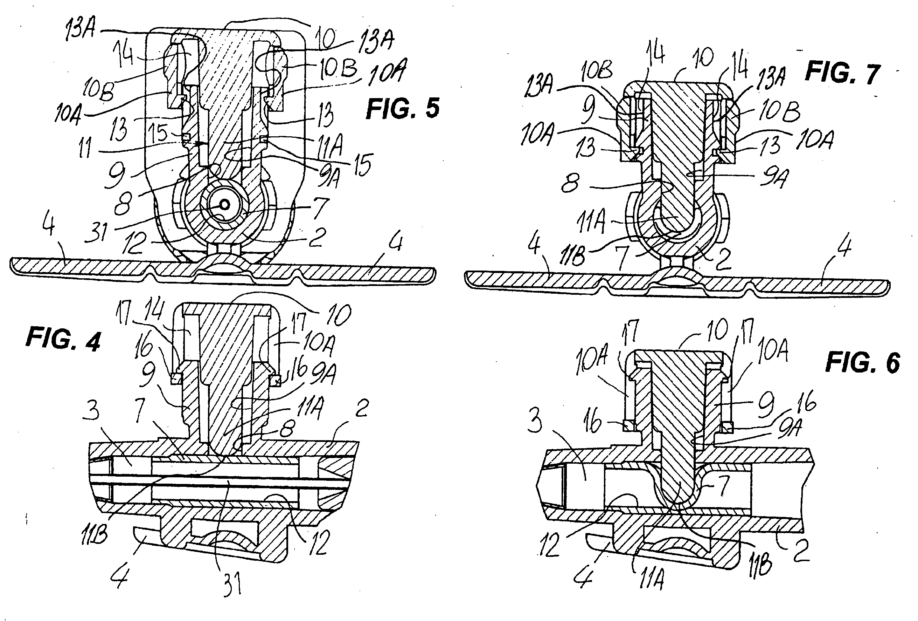

The closing member 11 may be axially moved between two positions: a first idle position (see Figures 4 and 5), in which it is completely lifted from the sleeve 7, and an operating position in which it is moved toward the sleeve, and presses it until it blocks its central lumen (see Figures 6 and 7).

The cap 10 and the mouthpiece 9 may be mutually coupled in two manners, but in both cases the cap 10 is used by the operators as a control member to cause the pin 11a to slide into the chamber 9A between its two positions.

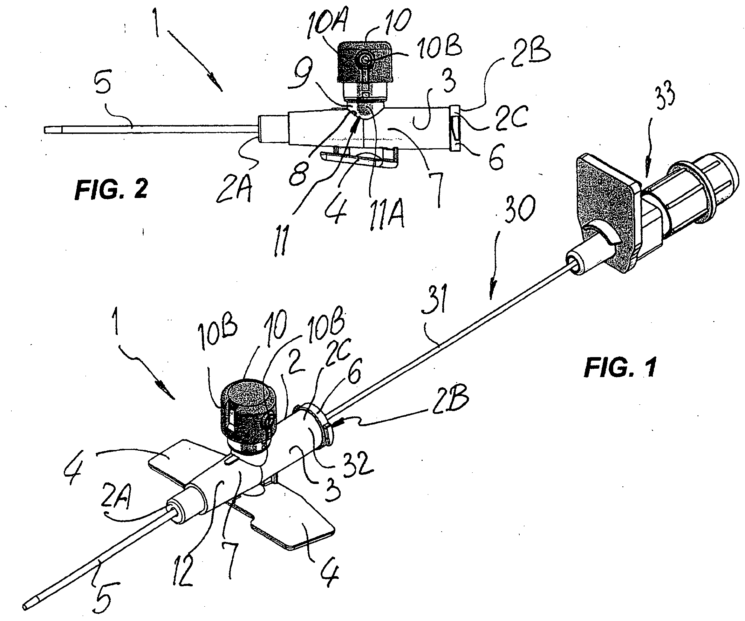

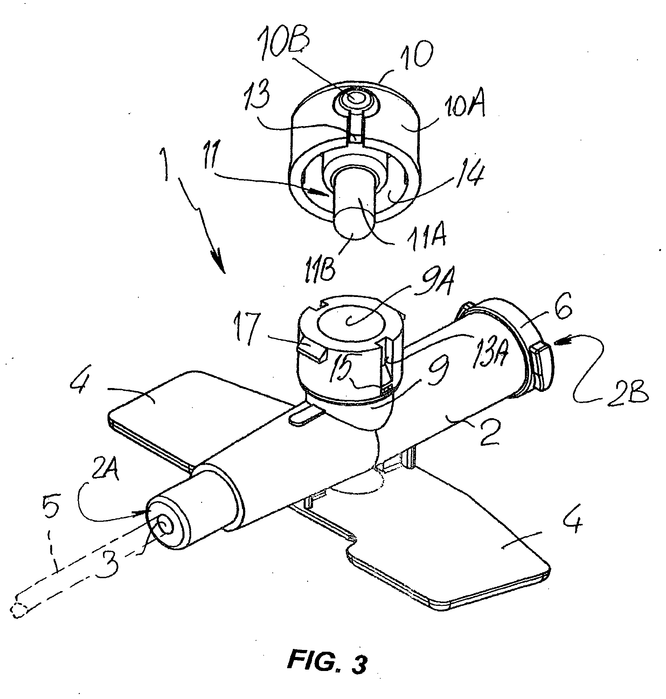

In a first example, as shown in Figures 1 to 8, the cap has a top face with a cylindrical ring 10A extending therefrom, with two bilaterally opposed buttons formed therein, referenced 10B.

The two buttons 10B are elastically compressible and form mutually facing retaining teeth 13 at their respective lower ends, which are slidably received in two respective guide grooves 13A formed in the lateral surface of the mouthpiece 9.

The ring 10A extends on the mouthpece 9, parallel thereto, and defines a gap 14 with the pin 11A, for the mouthpiece 9 to be received and slide therein relative to the cap 10 when the latter is moved toward the body 2, thereby also acting as a slide guide.

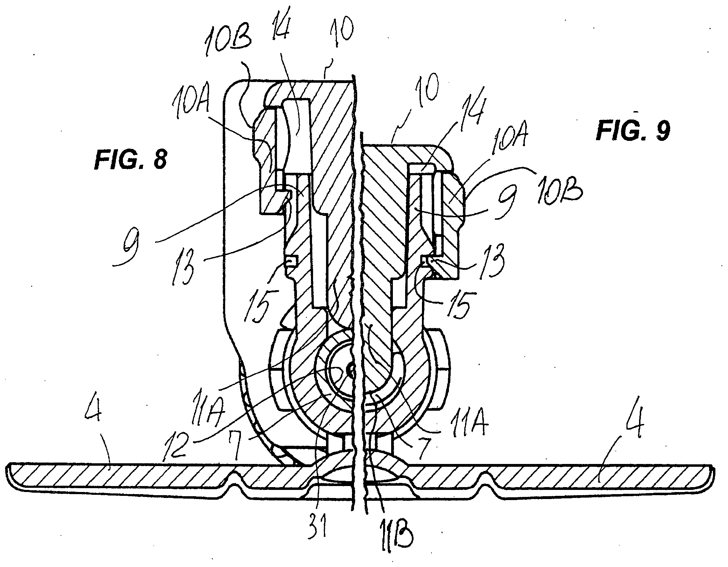

Two notches 15 are formed on the outer surface of the mouthpiece, for the retaining teeth 13 to engage therein, when the cap 10 is completely moved into the operating position of the pin 11A (see Figures 7 and 9).

The ring 10A forms at its base two segments of retaining edges 16 which are adapted to act as stop members for two corresponding additional teeth 17, that are formed at the free end of the mouthpiece 9 such that their engagement against the retaining edges 16 prevents the cap 10 from being pulled out beyond its free end.

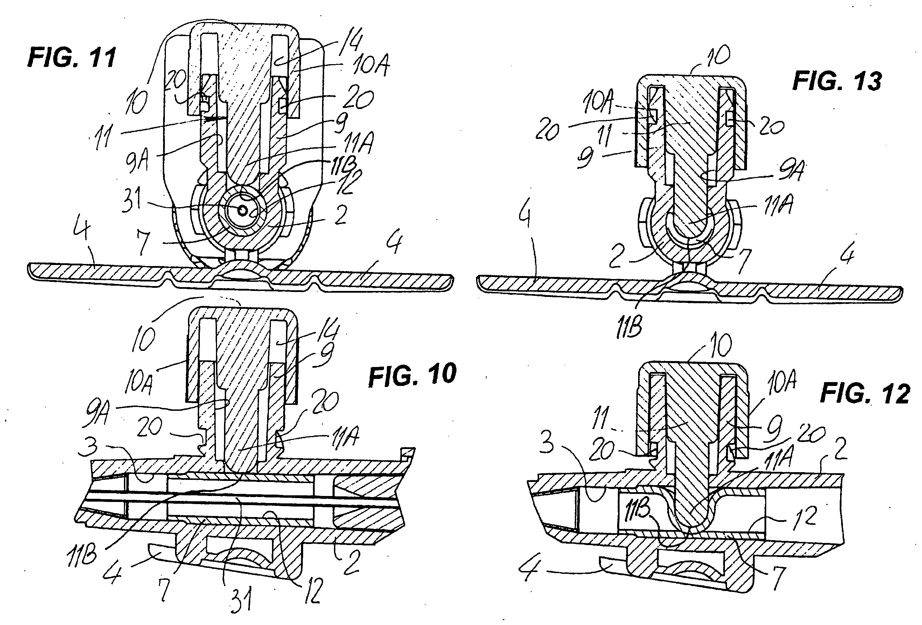

Referring now to Figures 10 to 14, an embodiment of the anti-reflux catheter is shown, and the following description of the embodiment uses the same references for the parts in common with the first example for ready understanding.

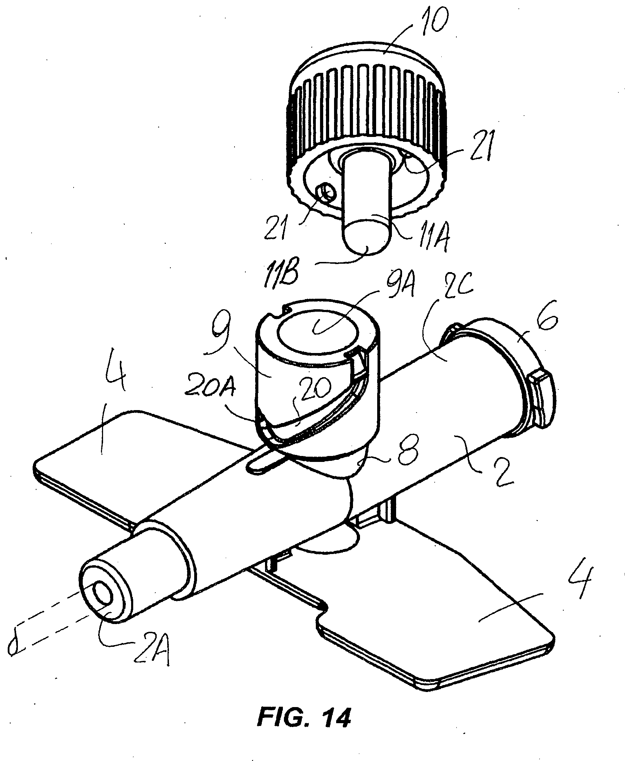

In greater detail, Figure 14 shows the main feature of this embodiment, i.e. a double helical groove 20 consisting of two sections formed on the outer surface of the mouthpiece 9 on opposite sides, and having respective straight end segments 20A slightly oriented upward, such that the thrust of the silicone cylinder 7 when it is deformed by pressing reduces the possibility that the cap may be rotated, and hence accidentally opened.

Each of these two groove sections 20 are designed to allow sliding guided engagement of two corresponding pawls projecting out of opposite sides of the inner wall of the cap 10.

As the cap 10 is pressed onto the mouthpiece 9 toward the body 2, these pawls are slidingly guided into the helical grooves 20, whose helical profile causes the cover 10 to rotate during such movements.

By this movement of the cap 10, the pin 11A slides in the chamber 9A and alternately assumes the two possible positions, i.e. the operating position in which it transversely presses down the sleeve 7 until it obstructs its central lumen 12 (see Figures 12 and 13) or the idle position in which it is completely lifted therefrom (see Figures 10 and 11).

Still with reference to Figure 1 it shall be noted that the anti-reflux catheter 1 can be introduced into a blood vessel of a patient, by means of an introducer needle 30 which has a shaft 31 designed to be coaxially inserted into the flexible tubular cannula 5 to temporarily impart stiffness to the latter, to allow it to extend through the walls of the blood vessel and be adequately introduced therein.

The shaft 31 is supported by a control handle 33 located at one proximal end facing away from the tip 32.

The operation is as follows; the catheter 1 is introduced into a blood vessel of a patient in a known manner, i.e. by an injection with the help of the introducer needle 30.

As soon as the catheter 1 has been properly positioned, the operator pulls the introducer needle 30 away from the cannula 5 and the central body 2 until it is entirely removed from the end 2A of the latter.

In order to prevent the blood in the blood vessel from flowing out of the catheter 1, the operator acts on the cap 10, by causing it to slide on the mouthpiece 9 it toward the body 2.

In the first example of the catheter, the operator typically presses the cap 10, and this will cause the pin 11A to move from the idle position to the operating position in which it crushes the sleeve 7, by sliding in the chamber 9A, until it occludes its central lumen 12 and prevents the passage of reflux blood from the blood vessel.

The cap 10 moves perpendicular to the body 2 and is guided by the teeth 13 which slide in the corresponding grooves 13A formed in the mouthpiece 9 almost along their entire travel.

As soon as the pin 11A reaches its final operating position, the teeth 13 engage in their respective notches 5 and hold the cap 10, and hence the pin 11A in the operating position in which they occlude the lumen 12 as long as is required by the operator to couple the end of a drug feeding tube to the connector 6 for infusion of the drug into the patient.

Once such connection is completed, the operator will press the two buttons 10B, which cause the teeth 13 to be disengaged from their respective notches 15, thereby allowing the cap 10 and the pin 11A to be lifted from the sleeve 7, and to clear the passage through the lumen.

This operation may be repeated if the operator has to disconnect a drug delivery tube and connect another tube for administration of a different drug.

In the embodiment of the catheter 1, after removal of the introducer needle 30 from the catheter 1 the operator prevents blood reflux by still acting on the cap 10, i.e. by screwing it down on the mouthpiece 9 such that it can be progressively lowered toward the body 2.

Such downward motion, like in the first example, causes the pin 11A to slide into the chamber 9A and to consequently progressively crush the sleeve 7 in the transverse direction until the lumen of the latter is completely obstructed, thereby preventing blood reflux from the vessel of the patient.

The screw motion of the cap 10 is allowed by engagement of the two pawls 21 in the corresponding helical grooves 20 and, when the pawls 21 reach the bottom end of the helical grooves 20 they are held by the upward end segments 20A thereof, whereby the cap 10 is retained in the operating position of the pin 11A.

While the pin 11A obstructs the lumen 12 of the sleeve 7, the operator may complete, as described above, the procedure for coupling the connector 6 to a concurrent end of a drug feeding tube, or may replace a tube with another, without any risk of outward reflux of patient blood.

When the connection or replacement of the tube has been completed, the operator acts on the cap 10 again to impart an unscrewing movement thereto, thereby causing the pin 11A to be lifted from the sleeve 7 and clearing the passage through the lumen 12 thereof.

The invention has been found to fulfill the intended objects.

The invention so conceived is susceptible to changes and variants within the inventive concept.

Also, all the details may be replaced by other technical equivalent elements.

In its practical implementation, any material, shape and size may be used as needed, without departure from the scope as defined by the following claims.

## Claims

1. An anti-reflux catheter comprising:
- A support center body (2) having an inner longitudinal passage chamber (3), briefly inner chamber (3) ;
- A cannula (5) prolonging from one end (2A) of the center body (2) and connected to the inner chamber (3);
- A transversal opening (8) crossing the center body (2) and communicating with the inner chamber (3);
- A prolonging extension (9) of the transversal opening (8) toward the outside;
- A flexible sleeve (7) longitudinally and coaxially fitted in the inner chamber (3) in correspondence with the transversal opening (8) and having an outer surface facing said transversal opening (8);
- pressing means (10, 11) acting on said sleeve (7) and movable between a deactivated position or an activated position, and vice versa, associated with said transversal opening (8), said pressing means (10, 11) being activated from the outside,
said pressing means comprising:
- A maneuvering member (10) slidingly coupled to said extension (9) and to be alternatively positioned between a deactivated position raised from said center body (2) or an activated position lowered toward said center body (2);
- A shutter (11) actuated by said maneuvering member (10) and slidingly received in a sliding chamber (9A) defined inside said extension (9), said shutter (11) having an active contact end (11B) with the outer surface of said sleeve (7) and an oppposing end connected to said maneuvering member (10 said maneuvering member being a plug (10) fitted on said extension (9), the anti-reflux catheter further comprising guide means (20, 21) provided for the plug (10) and the extension (9), said guide means being capable to guide the plug between a deactivated position and an activated position, said guide means comprising at least a coupling between a concave member (20) and a convex member (21) engaging said concave member (20);
said concave element comprising at least a groove (20) obtained in the outer surface of said extension (9) and said convex element comprises at least a pawl (21) extending from an inner surface of said plug (10), **characterized in that** said groove is a helical groove (20) wherein : temporary retention means (201) are provided for the helical groove and the plug, said temporary retention means being capable to retain the plug in the activated position; and wherein said temporary retention means comprise:
- At least one end segment (20A) of said helical groove (20);
the pawl (21) projecting from said plug (10) and removably engageable in said end segment (20A) when said plug (10) is in said activated position, said end segment (20A) being counter-sloped with respect of said helical groove (20).

## Patentansprüche

1. Katheter mit Rücklaufschutz, umfassend:
- einen Stützzentralkörper (2), der eine innere Längsdurchgangskammer (3), kurz innere Kammer (3), aufweist;
- eine Kanüle (5), die sich von einem Ende (2A) des Zentralkörpers (2) erstreckt und mit der inneren Kammer (3) verbunden ist;
- eine transversale Öffnung (8), die den Zentralkörper (2) durchquert und mit der inneren Kammer (3) kommuniziert;
- eine sich nach außen erstreckende Verlängerung (9) der transversalen Öffnung (8);
- eine biegsame Hülse (7) längswärts und koaxial eingepasst in die innere Kammer (3) in Übereinstimmung mit der transversalen Öffnung (8) und mit einer Außenfläche, die der transversalen Öffnung (8) zugewandt ist;
- Druckmittel (10, 11), die auf die Hülse (7) wirken und zwischen einer deaktivierten Position oder einer aktivierten Position und umgekehrt beweglich sind, zugehörig zu der transversalen Öffnung (8), wobei die Druckmittel (10, 11) von außen aktiviert werden,
wobei die Druckmittel umfassen:
- ein Manövrierbauteil (10), gleitend gekoppelt an die Verlängerung (9) und abwechselnd zwischen einer deaktivierten Position, angehoben von dem Zentralkörper (2), oder einer aktivierten Position, abgesenkt zu dem Zentralkörper (2), zu positionieren;
- einen Verschluss (11), betätigt durch das Manövrierbauteil (10) und gleitend aufgenommen in einer Gleitkammer (9A), die in der Verlängerung (9) definiert ist, wobei der Verschluss (11) ein aktives Kontaktende (11B) mit der Außenfläche der Hülse (7) und ein entgegengesetztes Ende, verbunden mit dem Manövrierbauteil (10), aufweist, wobei das Manövrierbauteil ein Stopfen (10) ist, der auf die Verlängerung (9) eingepasst ist,
wobei der Katheter mit Rücklaufschutz weitere Führungsmittel (20, 21) umfasst, vorgesehen für den Stopfen (10) und die Verlängerung (9), wobei die Führungsmittel in der Lage sind, den Stopfen zwischen einer deaktivierten Position und einer aktivierten Position zu führen,
wobei die Führungsmittel mindestens eine Kopplung zwischen einem konkaven Bauteil (20) und einem konvexen Bauteil (21), in Eingriff mit dem konkaven Bauteil (20), umfassen;
wobei das konkave Element mindestens eine Nut (20) umfasst, erhalten in der Außenfläche der Verlängerung (9) und wobei das konvexe Element mindestens eine Sperrklinke (21) umfasst, die sich von einer Innenfläche des Stopfens (10) erstreckt,
**dadurch gekennzeichnet, dass** die Nut eine schraubenförmige Nut (20) ist, wobei:
zeitweilige Rückhaltemittel (20A) für die schraubenförmige Nut und den Stopfen vorgesehen sind, wobei die zeitweiligen Rückhaltemittel in der Lage sind, den Stopfen in der aktivierten Position zurückzuhalten;
und wobei die zeitweiligen Rückhaltemittel umfassen:
- mindestens ein Endsegment (20A) der schraubenförmigen Nut (20);
wobei die Sperrklinke (21) von dem Stopfen (10) hervorsteht und in dem Endsegment (20A) beweglich im Eingriff ist, wenn der Stopfen (10) sich in der aktivierten Position befindet, wobei das Endsegment (20A) in Bezug auf die schraubenförmige Nut (20) gegenläufig geneigt ist.

## Revendications

1. Cathéter antireflux comprenant :
- un corps central de support (2) possédant une chambre interne (3) en forme de passage longitudinal, en raccourci une chambre interne (3) ;
- une canule (5) se prolongeant à partir d'une extrémité (2A) du corps central (2) et reliée à la chambre interne (3) ;
- une ouverture transversale (8) traversant le corps central (2) et communiquant avec la chambre interne (3) ;
- une extension de prolongement (9) de l'ouverture transversale (8) en direction de l'extérieur ;
- un manchon flexible (7) inséré en direction longitudinale et en direction coaxiale dans la chambre interne (3) en correspondance avec l'ouverture transversale (8) et possédant une surface externe opposée à ladite ouverture transversale (8) ;
- un moyen de pression (10, 11) agissant sur ledit manchon (7) et mobile entre une position désactivée ou une position activée, et vice versa, associé à ladite ouverture transversale (8), ledit moyen de pression (10, 11) étant activé depuis l'extérieur ;
ledit moyen de pression comprenant :
- un élément de manoeuvre (10) couplé en coulissement à ladite extension (9) et qui doit venir se disposer en alternance entre une position désactivée, surélevée par rapport audit corps central (2) ou une position activée, abaissée en direction dudit corps central (2) ;
- un obturateur (11) actionné par ledit élément de manoeuvre (10) et logé en coulissement dans une chambre coulissante (9A) définie à l'intérieur de ladite extension (9), ledit obturateur (11) possédant une extrémité de contact active (11B) avec la surface externe dudit manchon (7) et une extrémité opposée reliée audit élément de manoeuvre (10), ledit élément de manoeuvre représentant un bouchon (10) appliqué sur ladite extension (9) ;
le cathéter antireflux comprenant en outre un moyen de guidage (20, 21) prévu pour le bouchon (10) et l'extension (9), ledit moyen de guidage étant capable de guider le bouchon entre une position désactivée une position activée ;
ledit moyen de guidage comprenant au moins un accouplement entre un élément concave (20) et un élément convexe (21) venant s'engrener avec ledit élément concave(20) ;
ledit élément concave comprenant au moins une rainure (20) obtenue dans la surface externe de ladite extension (9) et ledit élément convexe comprenant au moins un cliquet (21) s'étendant à partir d'une surface interne dudit bouchon (10) ;
**caractérisé en ce que** ladite rainure est une rainure hélicoïdale (20) ;
dans lequel des moyens de rétention temporaire (20A) sont prévus pour la rainure hélicoïdale et le bouchon, lesdits moyens de rétention temporaire étant capables de retenir le bouchon dans la position activée ;
et dans lequel lesdits moyens de rétention temporaire comprennent :
- au moins un segment terminal (20A) de ladite rainure hélicoïdale (20) ;
le cliquet (21) faisant saillie par rapport audit bouchon (10) et étant à même de s'engrener de manière amovible dans ledit segment terminal (20A) lorsque ledit bouchon (10) se trouve dans ladite position activée, ledit segment terminal (20A) présentant une pente inversée par rapport à celle de ladite rainure hélicoïdale (20).
